# EUROPEAN PATENT APPLICATION

(11) **EP 4 361 274 A1**
(43) Date of publication of application: **01.05.2024**
(21) Application number: 22828505.2
(22) Date of filing: 23.06.2022
(51) Int. Cl.: C12N 15/53, C12N 1/15, C12N 1/19, C12N 1/21, C12N 5/10, C12N 9/02, C12N 15/63

(54) **LACCASE**

(30) Priority: 23.06.2021 JP 2021104265
(71) Applicant: Amano Enzyme Inc., Nagoya-shi Aichi 460-8630 (JP)
(72) Inventor: YACHI, Hiroyuki, Kakamigahara-shi, Gifu 509-0109 (JP); SAKAI, Kiyota, Kakamigahara-shi, Gifu 509-0109 (JP)
(74) Representative: Müller-Boré & Partner Patentanwälte PartG mbB
(86) International application number: PCT/JP2022/025137
(87) International publication number: WO 2022/270589

(57) **Abstract**

Provided is a laccase that exhibits excellent activity in pH ranges including the alkaline range. According to the present invention, a laccase having an amino acid sequence of a laccase derived from Paramyrothecium sp., and a laccase having a sequence that is similar thereto and that includes said amino acid sequence as the basic backbone exhibit excellent activity in pH ranges including the alkaline range.

## Description

### TECHNICAL FIELD

The present invention relates to a novel laccase.

### BACKGROUND ART

A laccase is an enzyme belonging to multicopper oxidase, and catalyzes a reaction in which a substrate is oxidized and oxygen is reduced by four electrons with remaining electrons to produce water. Examples of the substrate to be oxidized by the laccase typically include diphenols, and also include methoxy-substituted phenols and diamines. Since such catalytic ability can be applied to various chemical reactions, it is expected that the catalytic ability can be used in many industries. The laccase is known to exist in microorganisms, plants, and animals.

For example, Patent Document 1 describes a thermostable laccase derived from Trametes versicolor strain TV-1, and describes that it exhibits the best laccase activity at pH 2.

Patent Document 2 describes that the pH range in which laccases derived from Botrytis cinerea, Trametes versicolor, or other microbial sources and laccases that can be purchased from commercial sources and/or laccases produced using recombinant techniques exhibit activity is pH 3 to pH 7.

Patent Document 3 describes that the optimum pH of a laccase derived from a microorganism belonging to the genus Streptomyces is about 4.5, and the laccase has no activity at pH 6.5.

### PRIOR ART DOCUMENTS

### PATENT DOCUMENTS

Patent Document 1: International Publication No. 98/55628 A
Patent Document 2: Japanese Patent Laid-open Publication No. 2001-103989
Patent Document 3: Japanese Patent Laid-open Publication No. 2002-171968

### SUMMARY OF THE INVENTION

### PROBLEMS TO BE SOLVED BY THE INVENTION

As described above, the laccase is expected to be widely applied in the industry. The application mode also includes the case of catalyzing an oxidation reaction under alkaline conditions. However, at present, the working pH of a laccase produced on an industrial scale and put to practical use is in an acidic range to a weakly acidic range, and particularly in an alkaline range, the laccase cannot be used. As described above, in the conventional laccase, since the pH range in which activity is exhibited is limited, the laccase cannot sufficiently follow the application mode expected in the industry.

Therefore, an object of the present invention is to provide a laccase exhibiting excellent activity in a pH range including an alkaline range.

### MEANS FOR SOLVING THE PROBLEM

As a result of intensive studies, the present inventor has found, by chance, a laccase exhibiting activity in an alkaline range from a non-known strain library. The present invention has been completed based on this finding.

In other words, the present invention provides inventions of the following embodiments.

Item 1. A laccase including a polypeptide shown in any of the following (a) to (c):
(a) a polypeptide including the amino acid sequence shown in SEQ ID NO: 1;
(b) a polypeptide including an amino acid sequence in which one or several amino acids are substituted, added, inserted, or deleted in the amino acid sequence shown in SEQ ID NO: 1, and having laccase activity equivalent to that of the polypeptide shown in the above (a) in a pH range including an alkaline range; and
(c) a polypeptide including an amino acid sequence having 70% or more sequence identity to the amino acid sequence shown in SEQ ID NO: 1, and having laccase activity equivalent to that of the polypeptide shown in the above (a) in a pH range including an alkaline range.

Item 2. A DNA encoding the laccase according to item 1.

Item 3. An expression cassette or a recombinant vector containing the DNA according to item 2.

Item 4. A transformant obtained by transforming a host using the expression cassette or the recombinant vector according to item 3.

Item 5. A method for producing a laccase, the method including a step of culturing the transformant according to item 4.

Item 6. An enzyme preparation containing the laccase according to item 1.

Item 7. The enzyme preparation according to item 6, wherein the enzyme preparation is used as a protein crosslinking agent.

Item 8. The enzyme preparation according to item 6, which is used as an oxidation modifier for a food and drink or a food and drink material, an industrial material or an industrial waste component, or a pharmaceutical or scientific analysis material.

Item 9. A method for producing a crosslinked protein, the method including a step of allowing the laccase according to item 1 to act on a protein.

Item 10. The production method according to item 9, wherein the step is performed under an alkaline condition.

Item 11. The production method according to item 9 or 10, wherein in the step, a mediator selected from the group consisting of 3-(3,4-dihydroxyphenyl)alanine, catechin, and caffeic acid is used in combination with the laccase.

Item 12. A method for producing an oxidation-modified food and drink or food and drink material, industrial material or industrial waste component, or pharmaceutical or scientific analysis material, the method including a step of allowing the laccase according to item 1 to act on a food and drink or a food and drink material, an industrial material or an industrial waste component, or a pharmaceutical or scientific analysis material.

### ADVANTAGES OF THE INVENTION

The present invention provides a laccase exhibiting excellent activity in a pH range including an alkaline range.

### BRIEF DESCRIPTION OF THE DRAWINGS

Fig. 1 shows results of confirming crosslinking activity of a laccase derived from Paramyrothecium sp. to a pea protein under various pH conditions including a pH in an alkaline range.
Fig. 2 shows results of confirming crosslinking activity of a laccase derived from Paramyrothecium sp. to a soybean protein under various pH conditions including a pH in an alkaline range.
Fig. 3 shows results of confirming crosslinking activity of a laccase derived from Paramyrothecium sp. to a wheat protein under various pH conditions including a pH in an alkaline range.
Fig. 4 shows results of examining protein crosslinking activity when various mediators were used in combination with a laccase derived from Paramyrothecium sp.
Fig. 5 shows pH stability of a laccase derived from Paramyrothecium sp. (MM13-F2103).
Fig. 6 shows temperature stability of a laccase derived from Paramyrothecium sp. (MM13-F2103).
Fig. 7 shows optimum pH of a laccase derived from Paramyrothecium sp. (MM13-F2103).
Fig. 8 shows optimum temperature of a laccase derived from Paramyrothecium sp. (MM13-F2103).
Fig. 9 shows an appearance of a meat-like processed food product (derived from MM13) produced using a laccase derived from Paramyrothecium sp., in comparison with an appearance of a meat-like processed food product (without enzyme treatment) produced without using a laccase.

### EMBODIMENTS OF THE INVENTION

In the present description, a "non-polar amino acid" includes alanine, valine, leucine, isoleucine, proline, methionine, phenylalanine, and tryptophan. A "non-charged amino acid" includes glycine, serine, threonine, cysteine, tyrosine, asparagine, and glutamine. An "acidic amino acid" includes aspartic acid and glutamic acid. A "basic amino acid" includes lysine, arginine, and histidine.

### 1. Laccase

A laccase of the present invention includes a polypeptide shown in any of the following (a) to (c):
(a) a polypeptide including the amino acid sequence shown in SEQ ID NO: 1;
(b) a polypeptide including an amino acid sequence in which one or several amino acids are substituted, added, inserted, or deleted in the amino acid sequence shown in SEQ ID NO: 1, and having laccase activity equivalent to that of the polypeptide shown in the above (a) in a pH range including an alkaline range; and
(c) a polypeptide including an amino acid sequence having 70% or more sequence identity to the amino acid sequence shown in SEQ ID NO: 1, and having laccase activity equivalent to that of the polypeptide shown in the above (a) in a pH range including an alkaline range.

Hereinafter, the laccase including the polypeptides of (a) to (c) will be described in detail.

SEQ ID NO: 1 is an amino acid sequence of a laccase derived from Paramyrothecium sp.

The polypeptides of (b) and (c) are sequence-similar laccases having the amino acid sequence of the polypeptide of (a) as a basic backbone.

The modification of an amino acid to be introduced into the polypeptide of the above (b) may include only one kind of modification (e.g., substitution) among substitution, addition, insertion, and deletion, or may include two or more kinds of modifications (e.g., substitution and insertion). In the polypeptide of the above (b), the number of amino acids to be substituted, added, inserted, or deleted may be 1 or more or several, and is, for example, 1 to 10, preferably 1 to 8, 1 to 6, 1 to 5, or 1 to 4, more preferably 1 to 3, and particularly preferably 1 or 2 or 1.

In the polypeptide of the above (c), the sequence identity may be 70% or more, but is preferably 80% or more, more preferably 90% or more, still more preferably 95% or more, still more preferably 98% or more, still more preferably 99% or more, particularly preferably 99.5% or more, and most preferably 99.8% or more.

Here, in the polypeptide of the above (c), for example, the sequence identity to the amino acid sequence shown in SEQ ID NO: 1 is sequence identity calculated as compared with the amino acid sequence shown in SEQ ID NO: 1. In addition, the "sequence identity" refers to a value of identity of an amino acid sequence obtained by bl2seq program of BLAST PACKAGE [sgi32 bit edition, Version 2.0.12; available from National Center for Biotechnology Information (NCBI)] (Tatiana A. Tatsusova, Thomas L. Madden, FEMS Microbiol. Lett., Vol. 174, p247-250, 1999). Parameters may be set to Gap insertion Cost value: 11 and Gap extension Cost value: 1.

In the polypeptides of the above (b) and (c), amino acids at positions 126, 128, 166, 168, 429, 432, 434, 484, 485, 486, 490, and 495 in the amino acid sequence shown in SEQ ID NO: 1 are considered to contribute to activity, and thus it is desirable not to introduce substitutions or deletions into these sites.

When an amino acid substitution is introduced into the polypeptides of the above (b) and (c), a conservative substitution is mentioned as an embodiment of the amino acid substitution. In other words, in the polypeptides of (b) and (c), examples of the amino acid substitution introduced into the amino acid sequence shown in SEQ ID NO: 1 include a substitution with another non-polar amino acid if the amino acid before substitution is a non-polar amino acid, a substitution with another non-charged amino acid if the amino acid before substitution is a non-charged amino acid, a substitution with another acidic amino acid if the amino acid before substitution is an acidic amino acid, and a substitution with another basic amino acid if the amino acid before substitution is a basic amino acid.

When an amino acid addition is introduced into the polypeptides of the above (b) and (c), examples of an embodiment of the amino acid addition include addition of a methionine residue to the N-terminal, and addition of a tag for purification (e.g., a binding oligopeptide such as oligohistidine).

The polypeptides of the above (b) and (c) include not only polypeptides obtained by artificial mutations but also polypeptides generated by naturally occurring mutations (mutants or variants) based on individual differences or species differences in an organism from which the polypeptides are derived.

With respect to the polypeptides of the above (b) and (c), the "alkaline range" refers to a range having a pH of more than 8.5, preferably a pH of 9 or more.

In the present invention, the "laccase activity" refers to at least activity that catalyzes a reaction for producing a crosslinked protein when a protein is used as a substrate (i.e., protein crosslinking activity). Specifically, the protein crosslinking activity is measured as follows.

A protein material is suspended and mixed in a buffer having a pH in an alkaline range so as to be 15% (w/v), and centrifuged to obtain a supernatant as a protein solution. 100 µl of this protein solution, 97 µl of a buffer having a pH in an alkaline range, and 3 µl of 200 mM DL-catechin (as a mediator) are mixed to obtain a substrate solution. 20 µl of this substrate solution and 10 µl of an enzyme (a laccase is used so as to have a concentration of 1.7 µg/ml as a protein concentration) are mixed and reacted at 37°C for 18 hours. Thereafter, the reaction solution is diluted 10 times and subjected to SDS-PAGE. The degree of protein crosslinking activity can be evaluated by the amount of a macromolecular protein (i.e., a protein polymerized by crosslinking. It appears as a band at the origin.) in SDS-PAGE.

Further, "having laccase activity equivalent to that of the polypeptide shown in (a)" means that when a protein is used as a substrate, the amount of a crosslinked protein produced by an action of the polypeptides of (b) and (c) is 80 to 120% of the amount of a crosslinked protein produced by the polypeptide of (a).

### 2. DNA

A DNA encoding the laccase of the present invention (hereinafter, it may be referred to as "DNA of the present invention") can be appropriately prepared and designed by those skilled in the art according to the amino acid sequence of the laccase of the present invention.

### 2-1. Design of DNA

The base sequence of the DNA encoding the laccase of the present invention can be appropriately designed by those skilled in the art according to the amino acid sequence described in the laccase of the present invention.

Specifically, examples of the DNA encoding the laccase of the present invention include a DNA shown in any of the following (i) to (iii):
(i) a DNA including the base sequence shown in SEQ ID NO: 2;
(ii) a DNA including a base sequence of a DNA that hybridizes with a DNA including a base sequence complementary to the base sequence shown in SEQ ID NO: 2 under stringent conditions; and
(iii) a DNA including a base sequence having 70% or more homology to the base sequence shown in SEQ ID NO: 2.

Hereinafter, the DNAs of (i) to (iii) will be described in detail.

The base sequence shown in SEQ ID NO: 2 is a base sequence encoding the amino acid sequence shown in SEQ ID NO: 1.

The DNAs of (ii) and (iii) are sequence-similar DNAs having the base sequence of the DNA of (i) as a basic backbone.

In the DNA of the above (ii), "under stringent conditions" refers to conditions of incubating at 50°C to 65°C for 4 hours to overnight in 6 × SSC (1 × SSC is 0.15 M NaCl, 0.015 M sodium citrate, pH 7.0) containing 0.5% SDS, 5 × Denhartz's [0.1% bovine serum albumin (BSA), 0.1% polyvinylpyrrolidone, 0.1% Ficoll 400], and 100 µg/ml of salmon sperm DNA.

Hybridization under stringent conditions is specifically performed by the following method. That is, a nylon membrane on which a DNA library or a cDNA library is immobilized is prepared, and the nylon membrane is blocked at 65°C in a prehybridization solution containing 6 × SSC, 0.5% SDS, 5 × Denhartz's, and 100 µg/ml of salmon sperm DNA. Each probe labeled with ³²P is then added, and incubation is performed overnight at 65°C. This nylon membrane is washed in 6 × SSC at room temperature for 10 minutes, in 2 × SSC containing 0.1% SDS at room temperature for 10 minutes, and in 0.2 × SSC containing 0.1% SDS at 45°C for 30 minutes, and then autoradiography is performed, and a DNA specifically hybridized with the probe can be detected.

In the DNA of the above (iii), the above homology may be 70% or more, but is preferably 80% or more, more preferably 90% or more, still more preferably 95% or more, still more preferably 98% or more, still more preferably 99% or more, particularly preferably 99.5% or more, and most preferably 99.8% or more.

Here, the "homology" of a base sequence refers to a value of identity obtained by bl2seq program of BLAST PACKAGE [sgi32 bitedition, Version 2.0.12; available from the National Center for Biotechnology Information (NCBI)] (Tatiana A. Tatsusova, Thomas L. Madden, FEMS Microbiol. Lett., Vol. 174, 247-250, 1999). Parameters may be set to Gap insertion Cost value: 11 and Gap extension Cost value: 1.

In the DNAs of the above (ii) and (iii), at least any of the base sequences encoding a tag for purification (e.g., a binding oligopeptide such as oligohistidine) may be further added to the DNA of the above (i).

### 2-2. Preparation of DNA

The DNA of the present invention can be obtained, for example, by obtaining at least a region encoding any of the polypeptides of the above (a) to (c) by PCR or the like using a DNA encoding any of the polypeptides of the above (a) to (c) as a template. The DNA encoding the laccase of the present invention can also be artificially synthesized by a gene synthesis method.

In addition, when a specific mutation is introduced into a specific site of a base sequence, a mutation introduction method is known, and for example, a site-specific mutation introduction method of DNA or the like can be used. As a specific method for converting a base in a DNA, for example, a commercially available kit can also be used.

The base sequence of a DNA into which a mutation has been introduced can be confirmed using a DNA sequencer. Once the base sequence is determined, the DNA encoding the laccase can be obtained by chemical synthesis, PCR using a cloned probe as a template, or hybridization using a DNA fragment having the base sequence as a probe.

In addition, it is possible to synthesize a mutant form of the DNA encoding the laccase and having a function equivalent to that before mutation by a site-directed mutagenesis method or the like. In addition, in order to introduce a mutation into the DNA encoding the laccase, the introduction can be performed by a known method such as a Kunkel method, a Gapped duplex method, or a megaprimer PCR method.

The DNA of the present invention is preferably one in which the codon usage frequency is optimized for the host. For example, when Escherichia coli is used as a host, a DNA in which the codon usage frequency is optimized for Escherichia coli is suitable.

### 3. Expression cassette or recombinant vector

An expression cassette or recombinant vector containing the DNA encoding the laccase of the present invention (hereinafter, also referred to as "expression cassette of the present invention" or "recombinant vector of the present invention") includes the DNA encoding the laccase of the present invention. The expression cassette or recombinant vector of the present invention can be obtained by linking a promoter and a terminator to the DNA of the present invention, or by inserting the expression cassette of the present invention or the DNA of the present invention into an expression vector.

The expression cassette or recombinant vector of the present invention may further contain, as a control factor, a transcription element such as an enhancer, a CCAAT box, a TATA box, or an SPI site as necessary, in addition to a promoter and a terminator. These control factors may be operably linked to the DNA of the present invention. Operably linking means that various control factors that control the DNA of the present invention are linked to the DNA of the present invention in a state in which they can operate in a host cell. In addition, the expression cassette or recombinant vector of the present invention may be configured to further contain a protease recognition sequence and an N-terminal sequence and/or a C-terminal sequence.

As the expression vector, those constructed for genetic recombination from a phage, a plasmid, or a virus capable of autonomously replicating in a host are suitable. Such expression vectors are known, and those skilled in the art can appropriately select and use an appropriate combination with a host cell. For example, in the case of using a microorganism as a host, examples of the expression vector include pBluescript (pBS) II SK(-) (manufactured by Stratagene), pSTV-based vectors (manufactured by Takara Bio Inc.), pUC-based vectors (manufactured by Takara Bio Inc.), pET-based vectors (manufactured by Merck), pGEX-based vectors (manufactured by GE Healthcare), pCold-based vectors (manufactured by Takara Bio Inc.), pHY300PLK (manufactured by Takara Bio Inc.), pUB 110 (Mckenzie, T. et al., 1986, Plasmid 15(2), p.93-103), pBR322 (manufactured by Takara Bio Inc.), pRS403 (manufactured by Stratagene), and pMW218/219 (manufactured by NIPPON GENE CO., LTD.). In the case of using algae or microalgae as a host, examples of the expression vector include pUC19 (manufactured by Takara Bio Inc.), P66 (Chlamydomonas Center), P-322 (Chlamydomonas Center), pPha-T1 (see Yangmin Gong, et al., Journal of Basic Microbiology, 2011, vol. 51, p.666-672), and pJET1 (manufactured by Cosmo Bio Co., Ltd.). In the case of using a plant cell as a host, examples of the expression vector include pRI-based vectors (manufactured by Takara Bio Inc.), pBI-based vectors (manufactured by Clontech Laboratories, Inc.), and IN3-based vectors (manufactured by INPLANTA INNOVATIONS INC.).

### 4. Transformant

By transforming a host using the expression cassette or recombinant vector of the present invention, a transformant (hereinafter, it may be referred to as "transformant of the present invention") is obtained.

The host used for production of the transformant is not particularly limited as long as introduction of a gene is possible and it can autonomously replicate and can express a trait of the gene of the present invention, but suitable examples thereof include microorganisms including bacteria belonging to the genus Escherichia such as Escherichia coli, the genus Bacillus such as Bacillus subtilis, the genus Pseudomonas such as Pseudomonas putida, and the like; actinomycetes and the like; yeast and the like; and filamentous fungi and the like, but the host may be an animal cell, an insect cell, a plant cell, and the like, in addition to these. Among them, Escherichia coli is particularly preferable.

The host used for production of the transformant may be Paramyrothecium sp., which is a bacterium or a cell from which the laccase of the present invention is derived.

The transformant of the present invention can be obtained by introducing the expression cassette or recombinant vector of the present invention into a host. The place where the DNA of the present invention is introduced is not particularly limited as long as a target gene can be expressed, and may be on a plasmid or on a genome. Specific examples of the method for introducing the expression cassette of the present invention or the recombinant vector of the present invention include a recombinant vector method and a genome editing method.

Conditions for introducing the expression cassette or recombinant vector of the present invention into a host may be appropriately set according to the type of the host, and the like. When the host is a microorganism, examples of the conditions include a method using a competent cell by calcium ion treatment, an electroporation method, a spheroplast method, and a lithium acetate method. When the host is an animal cell, examples of the conditions include an electroporation method, a calcium phosphate method, and a lipofection method. When the host is an insect cell, examples of the conditions include a calcium phosphate method, a lipofection method, and an electroporation method. When the host is a plant cell, examples of the conditions include an electroporation method, an Agrobacterium method, a particle gun method, and a PEG method.

### 5. Method for producing laccase

The laccase of the present invention can be produced by culturing the transformant of the present invention. The laccase of the present invention can also be produced by culturing Paramyrothecium sp. itself (untransformed).

Conditions for culturing the transformant or the bacterium or the cell from which the laccase is derived of the present invention may be appropriately set in consideration of nutritional physiological properties of the host or the bacterium or the cell, and liquid culture is preferable. In the case of industrial production, aeration stirring culture is preferable.

The transformant or the bacterium or the cell from which the laccase is derived of the present invention is cultured, and the culture supernatant or the cultured bacterial body or the cultured cell is recovered by a method such as centrifugation of the culture solution. When the laccase of the present invention is accumulated in the cultured bacterial body or the cultured cell, the bacterial body or the bacterial cell is treated with a mechanical method such as ultrasonic waves or French press or a lytic enzyme such as lysozyme, and if necessary, solubilized by using an enzyme such as protease or a surfactant such as sodium dodecyl sulfate (SDS), whereby a water-soluble fraction containing the laccase of the present invention can be obtained.

In addition, by selecting an appropriate expression vector and host, the expressed laccase of the present invention may be secreted into the culture solution.

The culture solution, water-soluble fraction, or protease-treated product containing the laccase of the present invention obtained as described above may be subjected to purification treatment as it is, or may be subjected to purification treatment after the laccase of the present invention in the culture solution, water-soluble fraction, or protease-treated product is concentrated.

The concentration can be performed by, for example, concentration under reduced pressure, membrane concentration, salting-out treatment, a fractionation precipitation method with a hydrophilic organic solvent (e.g., methanol, ethanol, and acetone), or the like.

The purification treatment of the laccase of the present invention can be performed, for example, by appropriately combining methods such as gel filtration, hydrophobic chromatography, ion exchange chromatography, and affinity chromatography.

The laccase of the present invention thus purified may be pulverized by freeze drying, vacuum drying, spray drying, or the like as necessary.

### 6. Enzyme preparation

The laccase of the present invention can be provided in the form of an enzyme preparation. Therefore, the present invention also provides an enzyme preparation containing the laccase of the present invention described above.

The content of the laccase of the present invention described above in the enzyme preparation of the present invention is not particularly limited, and can be appropriately set within a range in which laccase activity is exhibited.

The enzyme preparation of the present invention may contain, in addition to the laccase of the present invention described above, other components to the extent that the effect of the present invention is not affected. Examples of the other components include other enzymes other than the laccase of the present invention described above, mediators, additives, and culture residues generated by the above production method.

The other enzymes can be appropriately determined according to their application, and examples thereof include amylase (α-amylase, β-amylase, glucoamylase), glucosidase (α-glucosidase, β-glucosidase), galactosidase (α-galactosidase, β-galactosidase), protease (acidic protease, neutral protease, alkaline protease), peptidase (leucine peptidase, aminopeptidase), lipase, esterase, cellulase, phosphatase (acid phosphatase, alkaline phosphatase), nuclease, deaminase, oxidase, dehydrogenase (other than the above active ingredients), glutaminase, pectinase, catalase, dextranase, transglutaminase, protein deamidase, and pullulanase. These other enzymes may be contained alone or in combination of two or more thereof.

Examples of the mediator include 3-(3,4-dihydroxyphenyl)alanine (DOPA), catechin, and caffeine. These mediators may be contained alone or in combination of two or more thereof.

The additive can be appropriately determined according to the application of the laccase of the present invention described above and the preparation form of the enzyme preparation, and examples thereof include excipients, buffers, suspending agents, stabilizers, preservatives, antiseptics, and physiological saline. Examples of the excipient include starch, dextrin, maltose, trehalose, lactose, D-glucose, sorbitol, D-mannitol, sucrose, glycerol, and pectin. Examples of the buffer include phosphate, citrate, and acetate. Examples of the stabilizer include propylene glycol and ascorbic acid. Examples of the preservative include phenol, benzalkonium chloride, benzyl alcohol, chlorobutanol, and methylparaben. Examples of the antiseptic include ethanol, benzalkonium chloride, paraoxybenzoic acid, and chlorobutanol. These additives may be contained alone or in combination of two or more thereof.

Examples of the culture residue include components derived from a culture medium, contaminant proteins, bacterial body components, and cell components.

The preparation form of the enzyme preparation of the present invention is not particularly limited, and examples thereof include a liquid form and a solid form (powder, granules, and the like). Enzyme preparations in these preparation forms can be prepared by generally known methods.

Specifically, the enzyme preparation of the present invention can be used as a protein crosslinking agent, and can also be used as an oxidation modifier for a food and drink or a food and drink material, an industrial material or an industrial waste component, or a pharmaceutical or scientific analysis material. Details of the object of the oxidation modification and the oxidation modification are as described in "7. Use of laccase" described below.

### 7. Use of laccase

The laccase of the present invention can be used in any application utilizing an oxidation reaction of a substrate and/or various accompanying chemical reactions caused by radical species of reaction intermediates generated by the oxidation reaction. Examples of such an oxidation reaction or an accompanying chemical reaction include oxidation of phenolic compounds such as o-,p-diphenol, urushiol, and laccol; oxidation of aromatic amines such as p-phenylenediamine; degradation of lignin; crosslinking of proteins having functional groups that are easily oxidized, such as a tyrosine side chain (phenolic hydroxy group), a cysteine side chain (sulfhydryl group), a lysine side chain (ε-amino group), and a histidine side chain (imidazole group). In the present description, chemically changing a substrate by such an oxidation reaction and accompanying chemical reaction of the laccase is also referred to as "oxidation modification".

### 7-1. Method for producing crosslinked protein

Among the embodiments of the oxidation modification described above, particularly preferably, changing by crosslinking of proteins is exemplified. Therefore, the present invention also provides a method for producing a crosslinked protein, the method including a step of allowing the laccase of the present invention described above to act on a protein.

In the method for producing a crosslinked protein of the present invention, from the viewpoint of further improving reaction efficiency, it is preferable to use mediators such as 3-(3,4-dihydroxyphenyl)alanine (DOPA), catechin, and caffeine in combination with the laccase in the step. These mediators may be contained alone or in combination of two or more thereof.

In the method for producing a crosslinked protein of the present invention, the reaction conditions (e.g., the amount of enzyme, reaction time, reaction pH, reaction temperature, and the like) between a laccase and a protein are not particularly limited as long as protein crosslinking is achieved to a desired degree, and can be appropriately set by those skilled in the art according to the type and/or concentration of the laccase of the present invention, the type and/or concentration of the protein, and/or the desired degree of protein crosslinking, and the like.

Since the laccase used in the method for producing a crosslinked protein of the present invention exhibits excellent laccase activity at least in an alkaline range, the reaction pH is preferably more than 8.5, and more preferably 9 or more. The upper limit of the reaction pH is, for example, 12 or less, preferably 11 or less, more preferably 10 or less, and still more preferably 9.5 or less.

In addition, since the laccase used in the method for producing a crosslinked protein of the present invention preferably exhibits excellent laccase activity not only in an alkaline range but also in a neutral range, the reaction pH may be, for example, 5 or more, 6 or more, 7 or more, or 8 or more. The upper limit of the reaction pH is, for example, 12 or less, preferably 11 or less, more preferably 10 or less, and still more preferably 9.5 or less, 9 or less, or 8.5 or less.

Preferable examples of the reaction temperature include 4 to 45°C, and more preferably 25 to 40°C.

### 7-2. Method for producing oxidation-modified food and drink or food and drink material, industrial material or industrial waste component, or pharmaceutical or scientific analysis material

Examples of the object of the oxidation modification described above include a food and drink or a food and drink material, an industrial material or an industrial waste component, or a pharmaceutical or scientific analysis material. Therefore, the present invention further provides a method for producing an oxidation-modified food and drink or food and drink material, industrial material or industrial waste component, or pharmaceutical or scientific analysis material, the method including a step of allowing the laccase of the present invention described above to act on a food and drink or a food and drink material, an industrial material or an industrial waste component, or a pharmaceutical or scientific analysis material.

Examples of the oxidation modification of a food and drink or a food and drink material include crosslinking of proteins, improvement in binding properties of a tissue-like plant protein, thickening or gelatinization of a food, browning treatment of black tea, and removal of bitter astringency of a food. Examples of the oxidation modification of an industrial material or an industrial waste component include production of artificial lacquer, removal of lignin from pulp, detoxification of a waste liquid containing highly toxic phenolic compounds and/or aromatic amines, synthesis of organic compounds, production of adhesives, and synthesis of concrete admixtures. Examples of the oxidation modification of a pharmaceutical or scientific analysis material include conversion of an inspection target component into a sensable component and crosslinking of an analysis target protein.

In the method for producing an oxidation-modified food and drink or food and drink material, industrial material or industrial waste component, or pharmaceutical or scientific analysis material of the present invention, from the viewpoint of further improving reaction efficiency, it is preferable to use mediators in combination with the laccase in the step. Examples of the mediators that can be used are as described in the above "7-1. Method for producing crosslinked protein".

In the method for producing an oxidation-modified food and drink or food and drink material, industrial material or industrial waste component, or pharmaceutical or scientific analysis material of the present invention, the reaction conditions (e.g., the amount of enzyme, reaction time, reaction pH, reaction temperature, and the like) between a laccase and an object of oxidation modification (i.e., a food and drink or a food and drink material, an industrial material or an industrial waste component, or a pharmaceutical or scientific analysis material) are not particularly limited as long as oxidation modification is achieved to a desired degree, and can be appropriately set by those skilled in the art according to the type and/or concentration of the laccase of the present invention, the type and/or concentration of the object of oxidation modification, and/or the desired degree of oxidation modification, and the like. Preferable examples of the reaction pH and the reaction temperature are as described in the above "7-1. Method for producing crosslinked protein".

### EXAMPLES

Hereinafter, the present invention will be specifically described with reference to Examples, but the present invention is not to be construed as being limited to the following Examples.

### Test Example 1: Culture and enzyme purification

### (1-1) Culture and filtration

A 500 ml shake flask containing 100 ml of the laccase-producing medium shown in Table 1 was inoculated with a 1 cm square section of a Paramyrothecium sp. strain MM13-F2103 (country of origin: Myanmar) colony grown on a potato dextrose agar medium at 30°C for 3 days, and reciprocating shaking culture (140 r/min) was performed at 30°C for 3 days. The culture solution was subjected to solid-liquid separation by centrifugation, the supernatant was then separated by filtration, and the collected filtrate was used as a crude enzyme solution.

**[Table 1]**

| | (g/L) |
|---|---|
| Glucose | 50 |
| Peptone | 5 |
| Yeast extract | 1 |
| KH₂PO₄ | 2 |
| K₂HPO₄ | 1 |
| MgSO₄-7H₂O | 0.2 |
| CuSO₄·5H₂O | 0.05 |
| | 121°C, 20 minutes |

### (1-2) Primary purification (anion chromatography)

The crude enzyme solution was concentrated by ultrafiltration, and then dialyzed against a 20 mM Na-phosphate buffer (pH 7.0) + 0.3 M NaCl to obtain an internal dialysate. This internal dialysate was subjected to HiTrap Q Fast Flow (I.D. × L = 1.6 × 2.5 cm; manufactured by Cytiva) equilibrated with the above buffer. The above column was washed with 50 ml of the same buffer, and then the laccase adsorbed to the column was eluted and fractionated by a linear NaCl concentration gradient (0.3 to 0.6 M, 50 ml). A part of each of the obtained fractions was subjected to confirmation of laccase activity (oxidation of ABTS and crosslinking of proteins) by the following method, and a fraction having both activities was recovered and used as a primary purified solution.

### (1-3) Secondary purification (gel filtration chromatography)

The primary purified solution was concentrated by ultrafiltration, and then dialyzed against a 20 mM Na-phosphate buffer (pH 7.0) + 0.5 M NaCl to obtain an internal dialysate. This internal dialysate was subjected to HiLoad 16/600 Superdex 200 pg (I.D. × L = 1.6 × 60 cm; manufactured by Cytiva), and fractions having activity were combined and dialyzed against a 20 mM Na-phosphate buffer (pH 7.0) to obtain a purified laccase solution. Purification was confirmed by SDS-PAGE and Native-PAGE.

### <Confirmation of laccase activity - oxidation of ABTS>

20 µl of a 50 mM solution of ABTS (2,2'-azino-bis-(3-ethylbenzothiazoline-6-sulfonic acid)) as a substrate, 160 µl of a 125 mM sodium phosphate buffer (pH 7.0), and 20 µl of the enzyme solution were added to a microplate and mixed, and the mixture was reacted at 37°C for 15 minutes, and the absorbance at 405 nm before and after the reaction was measured. The presence of laccase activity was confirmed by confirming oxidation of ABTS.

### <Confirmation of laccase activity - crosslinking of proteins>

The following operations were performed for each of the three plant protein powders in Table 2 described below. The plant protein powders were suspended in a 50 mM Na-phosphate buffer (pH 7.0) so as to be 15% (w/v) and well mixed, and then subjected to solid-liquid separation by centrifugation at 12,000 × g for 5 minutes to obtain a supernatant as a protein solution. 100 µl of this protein solution, 97 µl of a 50 mM Na-phosphate buffer (pH 7.0), and 3 µl of 200 mM DL-catechin (mediator) were mixed to obtain a substrate solution. 20 µl of this substrate solution and 10 µl of an enzyme (a laccase is used so as to have a concentration of 1.7 µg/ml as a protein concentration quantified using a DC protein assay (manufactured by Bio-Rad Laboratories, Inc.)) were mixed and reacted at 37°C for 18 hours. Thereafter, the reaction solution was diluted 10 times, and the applied amount shown in Table 2 was subjected to SDS-PAGE. Protein crosslinking activity was confirmed by the presence of a macromolecular protein (a protein polymerized by crosslinking. It appears as a band at the origin.) in SDS-PAGE.

### Test Example 2: Evaluation of enzyme properties

### (2-1) Evaluation of laccase activity (protein crosslinking activity)

For each of a purified laccase obtained from Paramyrothecium sp. strain MM13-F2103 and "Laccase Y120" (it is also referred to as LC-Y120) manufactured by Amano Enzyme Inc., which is a laccase for comparison, protein crosslinking activity at a predetermined pH (pH 3.0, 5.0, 7.0, and 9.0) with respect to each of the three plant proteins shown in Table 2 was evaluated.

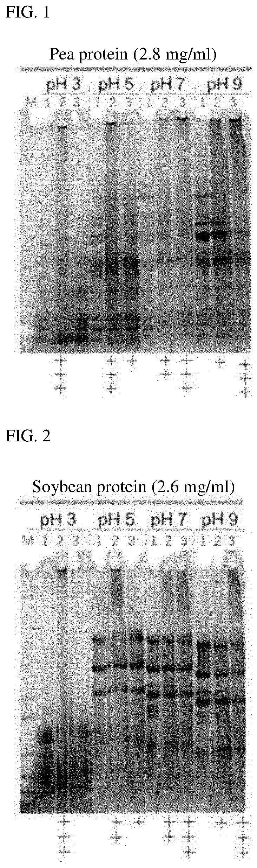

The following operations were performed for each of the three plant protein powders in Table 2. The plant protein powders were suspended in a buffer having a predetermined pH [50 mM Na-citrate buffer (pH 3.0 or 5.0), 50 mM Na-phosphate buffer (pH 7.0), or 50 mM Tris-HCl buffer (pH 9.0)] so as to be 15% (w/v) and well mixed, and then subjected to solid-liquid separation by centrifugation at 12,000 × g for 5 minutes to obtain a supernatant as a protein solution. 100 µl of this protein solution, 97 µl of the above buffer having a predetermined pH, and 3 µl of 200 mM DL-catechin (mediator) were mixed to obtain a substrate solution. 20 µl of this substrate solution and 10 µl of an enzyme (a laccase is used so as to have a concentration of 1.7 µg/ml as a protein concentration quantified using a DC protein assay (manufactured by Bio-Rad Laboratories, Inc.)) were mixed and reacted at 37°C for 18 hours. Thereafter, the reaction solution was diluted 10 times, and the applied amount shown in Table 2 was subjected to SDS-PAGE. Protein crosslinking activity was evaluated by visually observing the amount of a crosslinked protein (a protein polymerized by crosslinking. It appears as a band at the origin.) in SDS-PAGE based on the amount in the case of using a laccase (LC-Y120) for comparison. When the presence of the crosslinked protein was confirmed, the degree of the amount of the crosslinked protein was evaluated by the number of "+". The greater the number of "+" is, the greater the amount of crosslinked protein produced is. The results are shown in Figs. 1 to 3. In addition, the amount of the crosslinked protein can also be quantified using image analysis software such as imageJ.

**[Table 2]**

| Plant protein powder | Applied amount for SDS-PAGE |
|---|---|
| Pea protein powder (NUTRALYS S85F; manufactured by Roquette) | 10 µl |
| Soybean protein powder (SoyPro; manufactured by J-OIL MILLS, INC.) | 20 µl |
| Wheat protein powder (NUTRALYS W; manufactured by Roquette) | 20 µl |

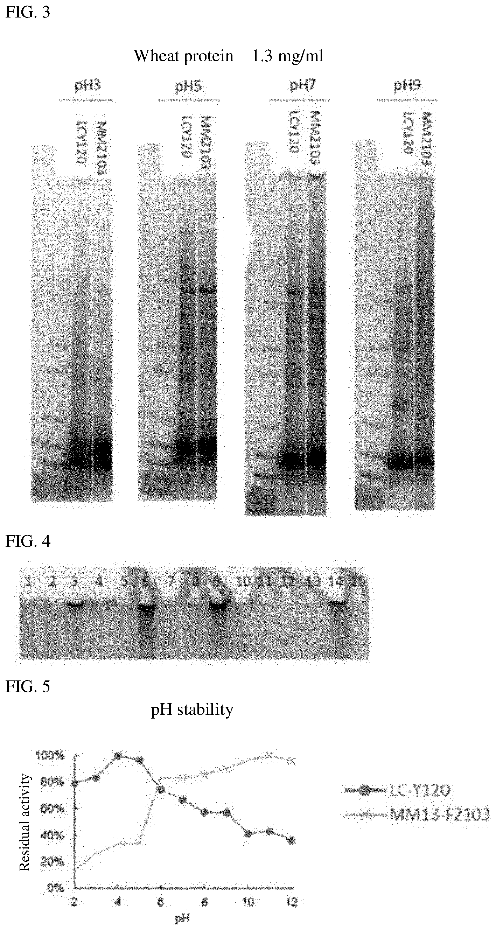

In Figs. 1 to 3, the leftmost lane (Lane 1 in Figs. 1 and 2 and the blank lane in Fig. 3) represents the result of a control in which no enzyme was added, the middle lane (Lane 2 in Figs. 1 and 2 and the lane of "LCY120" in Fig. 3) represents the result in the case of using the laccase for comparison, and the rightmost lane (Lane 3 in Figs. 1 and 2 and the lane of "MM2103" in Fig. 3) represents the result in the case of using the purified laccase obtained from Paramyrothecium sp. strain MM13-F2103.

As is clear from Figs. 1 to 3, for any plant protein, when the laccase LC-Y120 for comparison (Lane 2 in Figs. 1 and 2 and the lane of "LCY120" in Fig. 3) was used, almost no protein crosslinking activity was observed in the alkaline range (pH 9), whereas when the laccase obtained from Paramyrothecium sp. strain MM13-F2103 was used (Lane 3 in Figs. 1 and 2 and the lane of "MM2103" in Fig. 3), excellent protein crosslinking activity was observed in the alkaline range (pH 9). It was also found that the laccase obtained from Paramyrothecium sp. strain MM13-F2103 exhibits excellent protein crosslinking activity not only in the alkaline range but also in the ranges of pH 5 to 7, and thus has high versatility.

### (2-2) Evaluation of effect of promoting protein crosslinking by mediator

With respect to the laccase obtained from Paramyrothecium sp. strain MM13-F2103, the effect of promoting protein crosslinking by various mediators was evaluated by performing the same operation as in the above "(1-2) Method for evaluating laccase activity (protein crosslinking activity)" except that only the 50 mM Tris-HCl buffer (pH 9.0) was used, the mediator was changed to that described in Table 3 (however, it was used so as to have a concentration of 100 mM), the concentration of the laccase was used so as to be 33 µg/ml as a protein concentration, and the reaction time was set to 1 hour. The results are shown in Fig. 4. In Fig. 4, the number of each lane corresponds to the number in Table 3.

**[Table 3]**

| Lane No. | Mediator |
|---|---|
| 1 | Gallic acid monohydrate |
| 2 | Ferulic acid |
| 3 | Tannic acid |
| 4 | Vanillin |
| 5 | L-Tyrosine |
| 6 | L-DOPA |
| 7 | Eugenol |
| 8 | Isoeugenol |
| 9 | DL-Catechin |
| 10 | (±)-Limonene |
| 11 | L-Phenylalanine |
| 12 | L-Tryptophan |
| 13 | L-Cysteine hydrochloride monohydrate |
| 14 | Caffeic acid |
| 15 | MilliQ (control.) |

As is clear from Fig. 4, when L-DOPA, DL-catechin, and caffeic acid were used, the effect of promoting protein crosslinking was observed. Among these mediators, when L-DOPA and DL-catechin (particularly L-DOPA) were used, the effect of promoting protein crosslinking was remarkable.

### (2-3) pH stability

Each of the purified laccase obtained from Paramyrothecium sp. strain MM13-F2103 and the laccase LC-Y120 for comparison was dissolved in a 50 mM Britton-Robinson buffer having a different pH (pH 2 to 12), and the resulting enzyme solution was incubated at 4°C for 1 hour for pH treatment. For each enzyme solution, the same operation as in "<Confirmation of laccase activity - oxidation of ABTS>" in the above (1-3) was performed. The enzyme activity value was measured by defining enzyme activity that catalyzes oxidation of 1 µmol of ABTS per minute as 1 unit (U).

The relative value of the enzyme activity value after the pH treatment when the enzyme activity value at the treatment pH showing the highest residual activity was defined as 100% was derived as residual activity (%). The results are shown in Fig. 5. As is clear from Fig. 5, the laccase obtained from Paramyrothecium sp. strain MM13-F2103 had significantly improved stability in the alkaline range as compared with the commercially available laccase LC-Y120.

### (2-4) Temperature stability

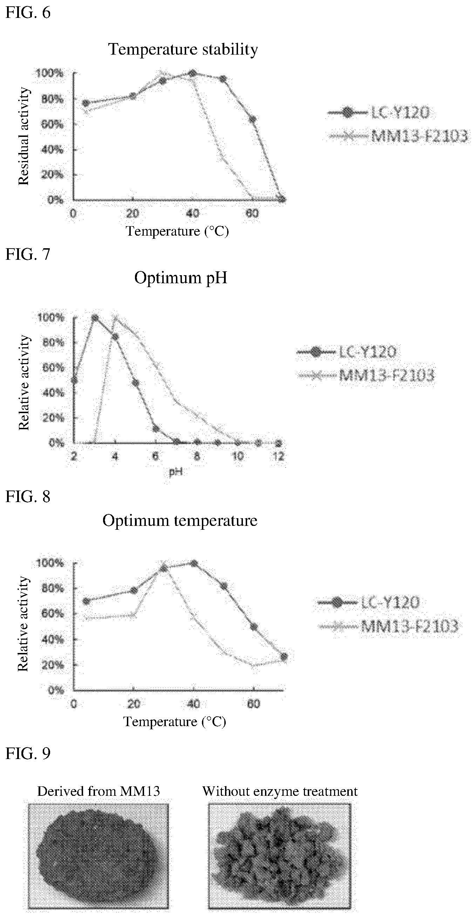

Each of the purified laccase obtained from Paramyrothecium sp. strain MM13-F2103 and the laccase LC-Y120 for comparison was dissolved in a 50 mM Britton-Robinson buffer (pH 7.0), and the resulting enzyme solution was incubated at 4°C, 20°C, 30°C, 40°C, 50°C, 60°C, 70°C, or 80°C for 1 hour for temperature treatment. For each of the enzyme solutions before and after the temperature treatment, the enzyme activity value was measured in the same manner as described in the above (2-3), and the relative value of the enzyme activity value after the temperature treatment when the enzyme activity value before the temperature treatment was defined as 100% was derived as residual activity (%). The results are shown in Fig. 6. As is clear from Fig. 6, the laccase obtained from Paramyrothecium sp. strain MM13-F2103 showed high stability at below 40°C.

### (2-5) Optimum pH

Using each of the purified laccase obtained from Paramyrothecium sp. strain MM13-F2103 and the laccase LC-Y120 for comparison, the same operation as in "<Confirmation of laccase activity - oxidation of ABTS>" in the above (1-3) was performed except that a 50 mM Britton-Robinson buffer having a different pH (pH 2 to 12) was used instead of the 50 mM Na-phosphate buffer (pH 7.0). The enzyme activity value was measured by defining enzyme activity that catalyzes oxidation of 1 µmol of ABTS per minute as 1 unit (U). The relative activity value when the enzyme activity value at the optimum pH of LC-Y120 was defined as 100% was derived. The results are shown in Fig. 7. As is clear from Fig. 7, the laccase obtained from Paramyrothecium sp. strain MM13-F2103 exhibited activity even in an alkaline range.

### (2-6) Optimum temperature

Using the purified laccase obtained from Paramyrothecium sp. strain MM13-F2103 and the laccase LC-Y120 for comparison, the same operation as in "<Confirmation of laccase activity - oxidation of ABTS>" in the above (1-3) was performed except that the reaction temperature was set to 4°C, 20°C, 30°C, 40°C, 50°C, 60°C, or 70°C instead of 37°C, and a 50 mM Britton-Robinson buffer having an optimum pH of each laccase was used instead of the 50 mM Na-phosphate buffer (pH 7.0). The enzyme activity value was measured by defining enzyme activity that catalyzes oxidation of 1 µmol of ABTS per minute as 1 unit (U). The relative activity value when the enzyme activity value at the optimum temperature of LC-Y120 was defined as 100% was derived. The results are shown in Fig. 8.

### Test Example 3: Sequence identification

Sequencing of a laccase obtained from Paramyrothecium sp. strain MM13-F2103 was performed. As a result, the sequence shown in SEQ ID NO: 3 (2018 bp) as a gene sequence, the sequence shown in SEQ ID NO: 2 (1764 bp) as a coding region sequence, and the sequence shown in SEQ ID NO: 1 (587 aa) as an amino acid sequence were determined.

### Test Example 4: Improvement in binding properties of tissue-like plant protein

Hot water (40°C) having a weight of 5 times was added to a granular soybean protein (manufactured by Marukome Co., Ltd.), and the mixture was left to stand for 10 minutes for swelling. Water was removed, and 25 g of the swollen granular soybean protein was weighed. To the swollen granular soybean protein, 2.75 g of a powdered pea protein (NUTRALYS F85M manufactured by Roquette) was mixed, and about 250 U (about 10 U per 1 g of swollen granular soybean protein) of a purified laccase obtained from Paramyrothecium sp. strain MM13-F2103 was added to prepare a soybean protein mixture. The soybean protein mixture was well mixed to make a hamburg steak-like form, and the formed mixture was left to stand at 25°C for 60 minutes. A meat-like processed food product was obtained through a firing step at 190°C for 15 minutes in an oven.

An appearance photograph of the obtained meat-like processed food product is shown in Fig. 9. Fig. 9 also shows, for comparison, a meat-like processed food product (without enzyme treatment) obtained in the same manner except that a laccase was not used. As shown in Fig. 9, binding was not observed at all in the case without enzyme treatment, whereas binding properties were observed in the case of treatment with the laccase derived from strain MM13.

### Test Example 5: Thickening of protein solution

The pea protein powder used in Test Example 2 was suspended in a buffer having a predetermined pH [50 mM Na-citrate buffer (pH 3.0 or 5.0), 50 mM Na-phosphate buffer (pH 7.0), or 50 mM Tris-HCl buffer (pH 9.0)] so as to be 15% (w/v), and this was used as a pea protein suspension. To this pea protein suspension, 0.1 mg/ml of a purified laccase obtained from Paramyrothecium sp. strain MM13-F2103 or laccase LC-Y120 for comparison in terms of protein weight and 5 mg/ml of protein glutaminase were added, and the mixture was reacted at 37°C for 18 hours under conditions of pH 3, 5, 7, or 9. Properties of the reaction product were evaluated as "-" when there was no change in physical properties and as "+" when thickening was observed. The results are shown in Table 4.

**[Table 4]**

| | pH3 | pH5 | pH7 | pH9 |
|---|---|---|---|---|
| LC-Y120 | - | - | - | - |
| MM13-F2103 | - | - | - | + |

As shown in Table 4, according to the purified laccase obtained from Paramyrothecium sp. strain MM13-F2103, thickening of the protein solution was observed by treatment in an alkaline range.

## Claims

1. A laccase comprising a polypeptide shown in any of the following (a) to (c):
(a) a polypeptide comprising the amino acid sequence shown in SEQ ID NO: 1;
(b) a polypeptide comprising an amino acid sequence in which one or several amino acids are substituted, added, inserted, or deleted in the amino acid sequence shown in SEQ ID NO: 1, and having laccase activity equivalent to that of the polypeptide shown in the above (a) in a pH range including an alkaline range; and
(c) a polypeptide comprising an amino acid sequence having 70% or more sequence identity to the amino acid sequence shown in SEQ ID NO: 1, and having laccase activity equivalent to that of the polypeptide shown in the above (a) in a pH range including an alkaline range.

2. A DNA encoding the laccase according to claim 1.

3. An expression cassette or a recombinant vector comprising the DNA according to claim 2.

4. A transformant obtained by transforming a host using the expression cassette or the recombinant vector according to claim 3.

5. A method for producing a laccase, the method comprising a step of culturing the transformant according to claim 4.

6. An enzyme preparation comprising the laccase according to claim 1.

7. The enzyme preparation according to claim 6, wherein the enzyme preparation is used as a protein crosslinking agent.

8. The enzyme preparation according to claim 6, which is used as an oxidation modifier for a food and drink or a food and drink material, an industrial material or an industrial waste component, or a pharmaceutical or scientific analysis material.

9. A method for producing a crosslinked protein, the method comprising a step of allowing the laccase according to claim 1 to act on a protein.

10. The production method according to claim 9, wherein the step is performed under an alkaline condition.

11. The production method according to claim 9, wherein in the step, a mediator selected from the group consisting of 3-(3,4-dihydroxyphenyl)alanine, catechin, and caffeic acid is used in combination with the laccase.

12. A method for producing an oxidation-modified food and drink or food and drink material, industrial material or industrial waste component, or pharmaceutical or scientific analysis material, the method comprising a step of allowing the laccase according to claim 1 to act on a food and drink or a food and drink material, an industrial material or an industrial waste component, or a pharmaceutical or scientific analysis material.
